# EUROPEAN PATENT SPECIFICATION

(11) **EP 4 574 016 B1**
(45) Date of publication and mention of the grant of the patent: **16.09.2026**
(21) Application number: 22955633.7
(22) Date of filing: 28.12.2022
(51) Int. Cl.: A61B 3/10, A61B 3/14, A61B 3/00

(54) **EXOPHTHALMOS-EVALUATING APPARATUS, METHOD AND SYSTEM FOR EXOPHTHALMOMETER**
EXOPHTHALMOS-EVALUIERUNGSVORRICHTUNG, VERFAHREN UND SYSTEM FÜR EXOPHTHALMOMETER
APPAREIL D'ÉVALUATION DE L'EXOPHTHALMIE, PROCÉDÉ ET SYSTÈME POUR EXOPHTHALMOMÈTRE

(30) Priority: 18.08.2022 CN 202210989502
(43) Date of publication of application: 25.06.2025
(73) Proprietor: Shangai Baiyi Healthcare Technology Co, Ltd, Shanghai 201203 (CN)
(72) Inventor: TIAN, Chaonan, Shanghai 201203 (CN); WANG, Youxiang, Shanghai 201203 (CN); WANG, Youzhi, Shanghai 201203 (CN); DU, Dong, Shanghai 201203 (CN)
(74) Representative: Elzaburu S.L.P.
(86) International application number: PCT/CN2022/142941
(87) International publication number: WO 2024/036866

(56) References cited:
- CN-A- 102 475 536
- CN-A- 104 720 738
- CN-A- 112 315 424
- CN-A- 112 806 956
- CN-A- 115 067 872
- CN-B- 107 960 985
- CN-U- 215 502 943
- CN-U- 216 823 409
- JP-A- 2020 054 784
- JP-B1- 5 950 007

## Description

### CROSS-REFERENCE TO RELATED APPLICATIONS

This application is a continuation of International Pat. Appl. No. PCT/CN2022/142941, filed on December 28, 2022, which claims priority to Chinese Pat. Appl. No. 202210989502.6, filed on August 18, 2022.

### TECHNICAL FIELD

The invention relates to the technical field of eye detection, in particular to an exophthalmos-evaluating apparatus and method for an exophthalmometer.

### BACKGROUND

Ocular prominence (eyeball protrusion degree) refers to the distance from the corneal vertex of the eyeball to the temporal orbital margin. The average ocular prominence of normal people is 12-14 mm, with an average of 13mm, and the difference between the two eyes is not more than 2 mm. However, patients with orbital diseases often have increased ocular prominence. At present, Hertel exophthalmos meter and CT scanning are the main methods for measuring ocular prominence in clinics.

Among them, the Hertel exophthalmos meter is a contact measurement, which requires manual recording of the degree during the experiment. The evaluation results between individuals are closely related to the experience of the diagnostic physician and the measurement method used, which is easy to lead to individual differences. Moreover, the close contact between patients and doctors in the clinical diagnosis and treatment environment increases the risk of infection exposure of both subjects and testers. In addition, some studies have suggested that when using Hertel exophthalmos meter for detection, because the transverse orbital edge is not completely symmetric, the degree of soft tissue collusion may vary, and there are no unified application standards or operating rules relating to the visual error and experience of the examiner, which will lead to significant differences in the readings of different examiners. Testers with significant experience tend to read 1 mm larger than inexperienced observers. Although the measurement accuracy of CT scans are higher than that in Hertel exophthalmos testing, there are problems such as large radiation doses, high measurement costs, and slow result generation. In view of the problems of low accuracy and high cost of the above methods, there are also solutions in the prior art that use automatic measuring equipment and methods to determine the ocular prominence. For example, as disclosed by Chinese patent no. CN104720738A (application number: 201510155640.4, publication date: June 24, 2015), the first distance X1 from a system reference point St of the ophthalmology equipment to the eye outer side frame edge Ek in the front and back direction, the second distance X2 from a system probe of the ophthalmology equipment to the system reference point St in the front and back direction, and the third distance X3 from the corneal vertex Ec to the system probe in the front and back direction are obtained, and the exophthalmic degree is obtained according to an equation Delta=X1+X2-X3. As described in Chinese patent no. CN112806956A (application number: 202110139034.9, public date: May 18, 2021), a range sensor is moved on a driver to identify the corneal vertex, and the distance between the light emitter and the range sensor is calculated to obtain the **ocular prominence.** The above methods all have the disadvantages of complex equipment and inaccurate corneal vertex recognitior

CN216823409U discloses a wearable eyeball protrusion (exophthalmos) measuring device comprising a rigid frame housing with laterally-movable brackets that abut the lateral orbital rims, carrying a pair of isosceles-right-triangle optical prisms and cameras, together with a pressure-belt control system that adjusts the brackets under constant pressure and image processing to automatically and reproducibly measure both orbital distance and eyeball protrusion.

### SUMMARY

In order to overcome the problems of large errors and high costs in the measurement of ocular prominence in the prior art, the invention provides an exophthalmos-evaluating apparatus and method for an exophthalmometer, aiming at improving the accuracy of the measurement of ocular prominence.

The first aspect of the invention provides an exophthalmos-evaluating apparatus for an exophthalmometer, which comprises:
A left displacement platform, a left canthal latch point, a left mirror, a camera, a near-infrared light source, a right canthal latch point, a right mirror, a right displacement platform, a visible light column and a housing; the left mirror and the right mirror are respectively arranged on the left canthal latch point and the right canthal latch point, the left canthal latch point and the right canthal latch point are respectively arranged on the left displacement platform and the right displacement platform, the left displacement platform and the right displacement platform are respectively arranged on both sides of the housing, and the left mirror and the right mirror are inclined; the camera is in front of the left mirror and the right mirror; the near-infrared light source is located below the camera; the visible light column is in multiple columns and is arranged in a longitudinal column along the inner side of the housing.

Furthermore, the apparatus includes two cameras, which are respectively located in front of the left mirror and the right mirror.

Furthermore, each column of the visible light columns includes one or more discrete point sources of light.

A second aspect of the present invention provides an exophthalmos-evaluating method, which may be based on the exophthalmos-evaluating apparatus described in the first aspect of the invention, and which comprises the following steps:
Step S1: Adjust and fix an lateral canthus, controlling the near-infrared light source to light up a plurality of columns of visible light columns in sequence,, and recording, by means of the camera, a video of the eyeball looking straight ahead,, and acquiring, from the video, a video frame when the longest reflected light of the visible light columns appears on the eyeball in the mirrors;
Step S2: Determining a corneal vertex from the video frame, and using a neural network to determine a pupil center;
Step S3: Calculating the ocular prominence on the basis of the known positions, the inclination angles of the mirrors, the corneal vertex, and the pupil center;
Wherein the known position is the position of the optical center of the camera, the distance between the optical center of the camera and the video frame, and the position of the lateral canthus (the outer canthus of the corner of the eye), and the inclination angle of the mirrors is the included angle between the mirror and the imaging surface.

Furthermore, in Step S1, to light up a plurality of columns of visible light columns in sequence, specifically meaning that the visible light columns are illuminated sequentially from the middle to the outside, or from the outside to the middle.

Furthermore, in Step S1, when the visible light column on one side is illuminated, a video of the opposite eye is captured.

Furthermore, in Step S3, the optical center is O, the outer canthus of the eye (the lateral canthus) is E, the distance between the optical center O and the video frame is OB, the imaging point of the corneal vertex Z on the video frame is C, and the reflection point U of the corneal vertex Z in the mirror is the imaging point A on the video frame, and the orthogonal distance from the imaging point C and the imaging point A to the optical axis are AB and CB respectively, and the inclination angle between the mirror and the eye is ∠ UED, and the distance from the optical center O to the face plane of the lateral canthus E is OF, and the distance between the lateral canthus E and the optical axis is EF; and the ocular prominence is ZG, which is the orthogonal distance between the corneal vertex Z and a straight line DE. The lengths of OB, OF, EF, AB, CB and the angle ∠ UED are known.

The steps to calculate the ocular prominence are as follows:
Step S31: The line passing through the reflection point U of the corneal vertex Z in the mirror and parallel to the line DE intersects the optical axis at a point W, intersects ZG at a point Y, and intersects the extended line of OC at point X;
Step S32: Find the length of line segment WF: $WF = \frac{OFtan ∠ UOW - EF}{tan ∠ UOW + \frac{1}{tan ∠ UED}}$
Step S33: Using iterative calculation to obtain the length of line segment ZY; and
Step S34: The ocular prominence is: $ZG = WF + ZY .$

According to another aspect of the invention, an evaluation system for implementing a method for evaluating exophthalmos as in the second aspect of the invention comprises:
A video acquisition module, configured to acquire the video of the eyeball looking straight ahead when the multiple visible light columns from the near-infrared light source light up in sequence;
An image acquisition module, configured to obtain, from the video, a video frame having the longest reflected light from the visible light column in the mirror;
A feature extraction module, configured to extract the corneal vertex and the pupil center from the video frame; and
A calculation module, configured to calculate the ocular prominence from the known position and the mirror inclination angle, the corneal vertex, and the pupil center, wherein the known position is the position of the optical center of the camera, the distance between the optical center of the camera and the video frame, and the position of the lateral canthus, and the inclination angle of the mirror is the included angle between the mirror and the imaging surface.

According to another aspect of the invention, a computer-readable storage medium storing at least one program code adapted to be loaded by a processor may perform the method of evaluating exophthalmos as in the second aspect of the invention.

Compared with the prior art, the technical scheme of the invention has at least the following beneficial effects:
By using the evaluating apparatus to capture eye video in the near infrared light field, and using a neural network recognition to effectively distinguish the iris, pupil and sclera are, the pupil center can be accurately identified. By setting a series of longitudinal visible light columns around the user's face and lighting up the visible light columns in sequence to find the longest reflected light of the visible light columns appearing on the eyeball in the mirror, the corneal vertex can be accurately and easily identified. After identifying the pupil center and the corneal vertex, combined with the known position and the inclination angle of the mirror, the algorithm of the present invention is used through the principle of optical imaging to accurately calculate the ocular prominence without contacting the cornea, so as to ensure accuracy and safety in the measurement of ocular prominence.

### BRIEF DESCRIPTION OF THE DRAWINGS

Fig. 1 is a stereogram of an exophthalmos-evaluating apparatus for an exophthalmometer in accordance with the invention;
Fig. 2 is a flow chart of the method of evaluating exophthalmos in accordance with the invention;
Fig. 3 is a video frame captured when the light reflected in the mirror is at its longest;
Fig. 4 is a diagram showing a basis or principle for calculating ocular prominence.
Fig. 5 is a diagram showing a structure of the evaluation system for exophthalmos.
1- Lower support frame; 2- Jaw support; 3- Left displacement platform; 4- Left canthal latch point; 5- Left mirror; 6- Forehead support; 7- Upper support frame; 8- Camera; 9- Near-infrared light source; 10- Right canthal latch point; 11- Right mirror; 12- Right displacement platform; 13- Visible light column; 14- Housing; 501- Video capture module; 502- Image acquisition module; 503- Feature extraction module; 504- Computing module.

### DETAILED DESCRIPTION OF THE PREFERRED EMBODIMENTS

Specific embodiments of the invention are further described in detail in combination with the attached drawings and examples. The following embodiments are used to illustrate the invention, but not to limit the scope of the invention.

It should be noted that OB, AB, CB, EF, etc. used in calculations in the present invention all refer to a corresponding line segment or its length.

### Example 1:

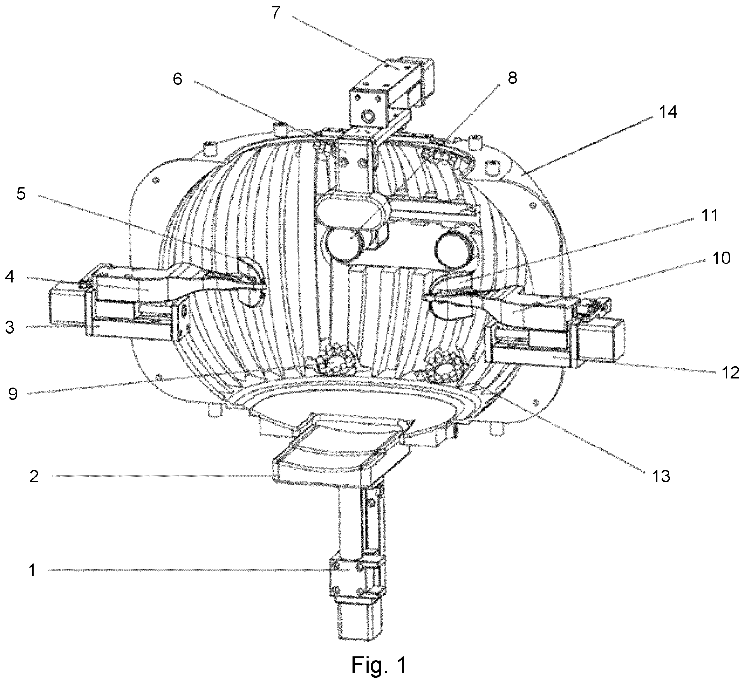

The present embodiment shown in Fig. 1 provides an exophthalmos-evaluating apparatus and method for an exophthalmometer, including a left displacement platform 3, a left canthal latch point 4, a left mirror 5, cameras 8, a near-infrared light source 9, a right canthal latch point 10, a right mirror 11, a right displacement platform 12, a visible light column 13, and a housing 14. The left mirror 5 and the right mirror 11 are respectively configured on the left canthal latch point 4 and the right canthal latch point 10, and the left canthal latch point 4 and the right canthal latch point 10 are respectively on the left displacement platform 3 and the right displacement platform 12; and the left displacement platform 3 and the right displacement platform 12 are respectively on both opposite sides of the housing 14, and the left mirror 5 and the right mirror 11 are inclined; and the cameras 8 are configured in front of the left mirror 5 and the right mirror 11; and the near-infrared light source 9 is configured below the camera 8; and the visible light column 13 is a multiple of columns and is configured longitudinally along an inner side of the housing 14.

In order to further support and adjust the evaluation apparatus and to ensure that the evaluation apparatus is positioned fixedly to the user, the evaluation apparatus also includes a lower support frame 1, a jaw support 2, a forehead support 6 and an upper support frame 7, wherein the lower support frame 1 and the upper support frame 7 are configured at the lower and upper parts of the housing 14, respectively, for supporting and rotating the entire evaluation apparatus, and the jaw support 2 and the forehead support 6 are configured to fix the user.

Furthermore, the cameras 8 can be provided in two numbers, which are respectively located in front of the left mirror 5 and the right mirror 11, so as to respectively take videos of the left and right eyes. In order to provide a uniform near-infrared light field, the near-infrared light source 9 can be configured as multiple light sources, evenly distributed under the camera 8. Each longitudinal visible light column 13 can be configured as an array of point-like light sources, and is externally wrapped in silicone.

When the apparatus is used to measure ocular prominence, the left displacement platform 3 and the right displacement platform 12 are first adjusted, and the lateral canthus points of each of the two eyes of the user are fixed using the left canthal latch point 4 and the right canthal latch point 10, to adapt to the facial features of different users. After fixing the user's eyes and face in position, the near-infrared light source 9 is turned on, and a series of visible light columns 13 configured orthogonally are turned on from the middle to the outside or from the outside to the middle. The visible light columns 13 will successively reflect light of different lengths on the eyeball in the mirror. The video frame in which the visible light column 13 forms the longest projection on the cornea through the corneal vertex can be found in the video taken. Through the video frame taken in the light field of the near-infrared light source 9, the pupil center can be accurately found by neural network analysis. The corneal vertex and pupil center, combined with the known distance and the mirror inclination angle, are used to calculate the ocular prominence.

### Example 2:

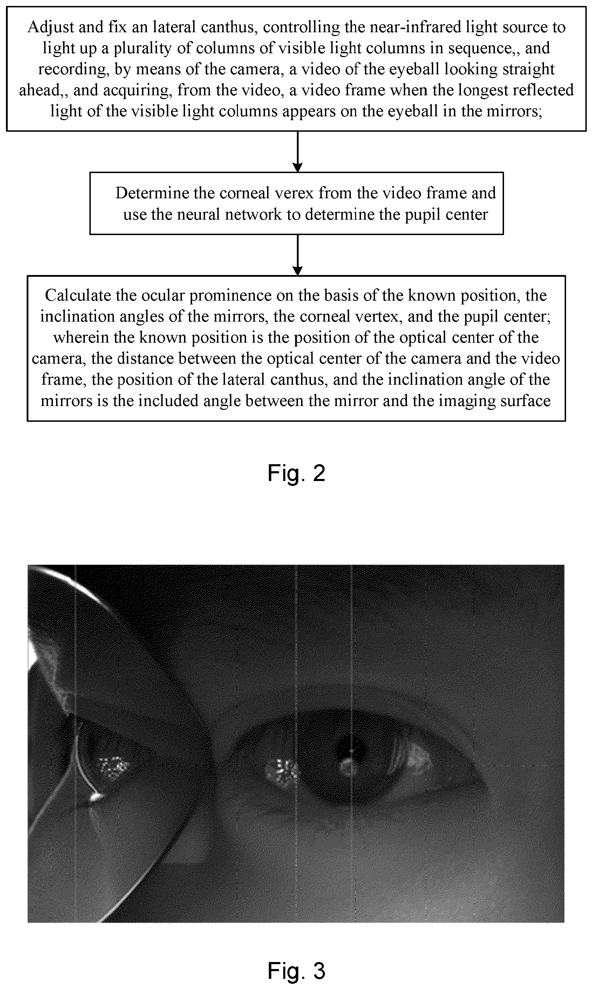

As shown in Figs. 2 and 3, this example provides a method for evaluating exophthalmos, which is illustrated below in conjunction with the exophthalmos-evaluating apparatus for an exophthalmometer. The method for evaluating exophthalmos involves the following steps:
Step S1: Adjust and fix the lateral canthus, make multiple visible light columns light up successively and control the near-infrared light source, capture the video of the eyeball looking straight ahead through the camera, and obtain the video frame of the longest reflected light of the visible light columns on the eyeball in the mirror from the video.

In Step S1 above, when the longitudinal visible light column on one side emits light, the video of the opposite eye is taken. Specifically, when a left visible light column 13 lights up, the video of the right eye is taken, and when a right visible light column 13 lights up, the video of the left eye is taken. Due to the setup of a series of longitudinal visible light columns 13, and the left mirror 5 and the right mirror 11 inclination angles, the visible light columns 13 are turned on successively from the outside to the inside, so that the visible light columns 13 present a series of reflected light of different lengths on the side of the eye in the mirror, and the longest projection of light from the visible light columns 13 on the cornea can be found through the reflected light on the eye in the mirror. For example, when the rightmost visible light column 13 emits light, the light reflected from the left eye on the side of the eye in the left mirror 5 is close to the inner canthus of the eye. As the light is emitted successively from visible light columns 13 towards the middle, the light from the light column 13 reflected in the side of the eye in the left mirror 5 gradually becomes longer until the reflected light passes through the corneal vertex, at which time the reflected light is the longest. The video frame at this point is obtained from the video.

Step S2: Determine the corneal vertex from the video frame and use the neural network to determine the pupil center.

As shown in Fig. 3, the corneal vertex can be determined by the video frame having the longest reflected light from the eyeball in the mirror. The corneal vertex is the tangent point of the reflected light on the eyeball in the left mirror and the orthogonal line in Fig. 3. In the normal visible light band of 400-700 nm, the color of different parts of the eye, namely the pupil, iris, and sclera, has little effect on imaging, due to the gradual structure of the corneal limbus in the contact part of the iris and sclera, resulting in the inability to accurately identify the spherical center of the eye. The peak absorption of human melanin pigment occurs at about 335 nm, and it is almost completely non-absorbed for wavelengths over 700 nm, while the reflectivity of iris is quite stable in near-infrared wavelengths over 700 nm. Therefore, the invention adopts a near-infrared light field, which can clearly distinguish the sclera, iris and pupil boundaries, and can accurately identify the pupil center in combination with a neural network training model. In one implementation, the RITnet neural network model is used in this example.

The exophthalmos-evaluating apparatus provided in Example 1 for exophthalmos testing also provides the ability to determine the pupillary center by manual operation after determining the corneal vertex from the video frame, which the user can select on demand with the neural network or by manual operation.

Step S3: Calculating the ocular prominence on the basis of the known positions, the inclination angles of the mirrors, the corneal vertex, and the pupil center.

In the above Step S3, the known position is the position of the optical center of the camera, the distance between the optical center of the camera and the video frame, and the position of the lateral canthus, and the inclination angle of the mirror is the included angle between the mirror and the imaging surface. The distance, obtained through the known position, refers to the distance between the optical center of the camera and the imaging surface (that is, the sensor), which is called the image distance in photography. The ocular prominence can be calculated by combining the above-mentioned distance, the inclination angle of the mirror with the imaging point of the pupil center and corneal vertex on the video frame.

### Example 3:

Based on Example 2 of the above method, this Example 3 further explains how to perform the mathematical calculation of ocular prominence.

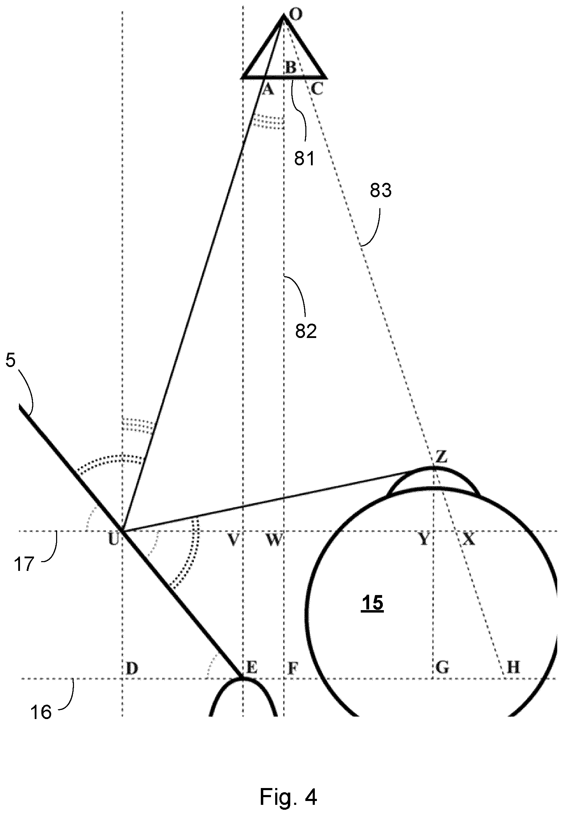

As shown in Fig. 4, the optical center is O, the lateral canthus is E, the orthogonal distance between the optical center O and the video frame is OB, the imaging point of the corneal vertex Z on the video frame is imaging point C, the imaging point of the reflection point U of the corneal vertex Z in the mirror on the video frame is the imaging point A, and the orthogonal distance from imaging point C and imaging point A to the optical axis are AB and CB, respectively. The angle of inclination between the mirror and the eye is ∠ UED, the distance between the optical center O and the face plane where the lateral canthus E is located is OF, the distance between the lateral canthus E and the optical axis is EF, and the ocular prominence is the orthogonal distance ZG from the corneal vertex Z to the straight line DE. The lengths of OB, OF, EF, AB and CB and the angle ∠ UED are known.

The steps to calculate the ocular prominence are as follows:
Step S31: The straight line passing through the reflection point U of the corneal vertex Z in the mirror and parallel to the straight line DE intersects with the optical axis at point W, with ZG at point Y, and with the extension line of the straight line OC at point X.

According to principles of imaging, both the imaging point C of the corneal vertex Z and the imaging point A of reflection point U of the corneal vertex Z in the mirror are in the video frame. The distance from CB and AB of imaging point C and imaging point A to the optical axis can be read in the video frame. According to principles of light reflection, the incidence angle of the corneal vertex Z in the mirror is equal to the reflection angle, namely: $∠ UOW + \left(\frac{Π}{2}-∠UED\right) = ∠ ZUY + ∠ UED$

This leads to: $∠ ZUY = \frac{Π}{2} + ∠ UOW - 2 ∠ UED$

Step S32: Given the length of OB, OF, AB, CB, EF and the size of ∠ UED, the following can be obtained according to analytic geometry: $tan ∠ UOW = \frac{AB}{OB}$ $tan ∠ WOX = \frac{CB}{OB}$ $\left\{\begin{matrix}OW + WF = OF \\ \frac{UW}{OW} = tan ∠ UOW \\ \frac{WF}{UW - EF} = tan ∠ UED\end{matrix}\right.$

Solved: $WF = \frac{OFtan ∠ UOW - EF}{tan ∠ UOW + \frac{1}{tan ∠ UED}}$ $OW = OF - WF$ $UX = OW \left(tan∠UOW+tan∠WOX\right)$

Step S33: Assume UY ≈UX, then $\hat{ZY} \approx UXtan ∠ ZUY$, where $\hat{ZY}$ is the estimated length of line segment ZY, and $\hat{YX}$ is the estimated length of line segment YX.

According to analytic geometry, the following can be obtained: $\left\{\begin{matrix}\hat{YX} = \hat{ZY} tan ∠ WOX \\ \hat{ZY} = \left(UX-\hat{YX}\right) tan ∠ ZUY\end{matrix}\right.$

Formula (7) is calculated iteratively until the error between two calculations of $\hat{ZY}$ are less than the error set value, at which time the iteration ends, and the following is taken: $ZY = \hat{ZY}$

Step S34: The ocular prominence is the distance ZG from the corneal vertex Z to the straight line DE: $ZG = WF + ZY$

The ocular prominence is calculated according to formula (4) and formula (8).

It can be seen that in order to calculate the ocular prominence, the length of line segment YG and line segment ZY in Fig. 4 should be calculated first. Since the length of line segment YG is equal to the length of line segment WF, the ocular prominence can be calculated by the sum of the distances of line segment WF and line segment ZY.

In Step S32, the length of line segment WF and the distance of line segment UX can be obtained by solving the ternary first order equation of formula (3). In Step S33, it is assumed that UY is equal to UX, that is, YX is equal to zero. In this case, the estimated value of ZY can be solved by trigonometric functions, that is, $\hat{ZY}$ and the value of $\hat{YX}$ can be calculated by $\hat{ZY}$, and then the value of $\hat{ZY}$ can be calculated by $\hat{YX}$, through iterative calculation until $\hat{ZY}$ converges. That is, when the error value calculated for $\hat{ZY}$ is less than the set value twice, then the iteration ends, that is, the length of line segment ZY is considered to be the final calculation result of $\hat{ZY}$*.*

The above calculation process can be automatically calculated by computer programming. By obtaining the video frame having the longest reflected light from the visible light columns in the mirror, input to the computer, one can automatically output the result of ocular prominence.

### Example 4:

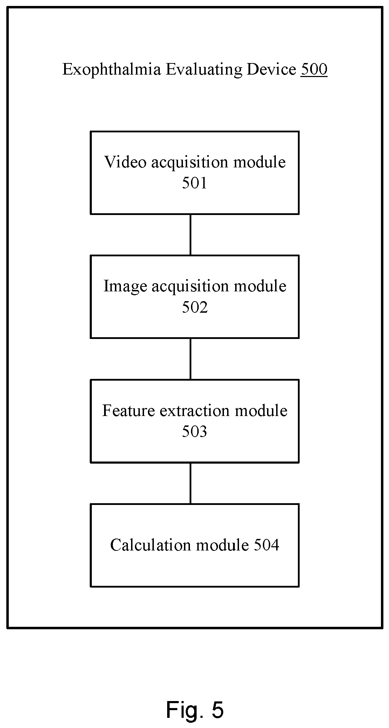

Fig. 5 is a structural diagram of an exophthalmos evaluation system provided by an embodiment of the invention, as shown in Fig. 5. The system comprises:
A video acquisition module 501, configured to acquire the video of the eyeball looking straight ahead when the multiple visible light columns from the near-infrared light source light up in sequence;
An image acquisition module 502, configured to obtain, from the collected video, the video frame having the longest reflected light from the visible light column in the mirror;
A feature extraction module 503, configured to determine the corneal vertex from the video frame acquired by the image acquisition module, and to extract the pupil center using a neural network; and
A calculation module 504, configured to calculate the ocular prominence through each parameter of the known position and the mirror inclination angle, the cornea vertex and the pupil center; wherein the known position comprises the position of the optical center of the camera, the distance between the optical center of the camera and the video frame and the position of the lateral canthus; and the inclination angle of the mirror is the included angle between the mirror and the imaging surface.

In an exemplary embodiment, a computer-readable storage medium is also provided, including a memory storing at least one piece of program code loaded and executed by a processor to complete an exophthalmos-evaluating method in the above embodiments. For example, the computer-readable storage medium may be a Read-Only Memory (ROM), a Random Access Memory (RAM), a compact disc (Compact Disc Read-Only Memory, CDROM), magnetic tape, floppy disk, an optical data storage device and the like.

A person of ordinary skill in the art may understand that all or part of the steps to implement the above embodiments may be performed by hardware or by hardware associated with at least one program code, which may be stored in a computer-readable storage medium, such as a read-only memory, a disk or an optical disc.

The above description is only a description of embodiments of the present invention, and is not intended to limit the present invention, and any modification, equivalent replacement, improvement, etc. made within the principles of the invention shall be included in the scope of protection of the invention.

## Claims

1. A exophthalmos-evaluating apparatus for an exophthalmometer, wherein comprising a left displacement platform (3), a left canthal latch point (4), a left mirror (5), cameras (8), a near-infrared light source (9), a right canthal latch point (10), a right mirror (11), a right displacement platform (12), visible light columns (13), and a housing (14), the left mirror (5) and the right mirror (11) are respectively set on the left canthal latch point (4) and the right canthal latch point (10), and the left canthal latch point (4) and the right canthal latch point (10) are respectively set on the left displacement platform (3) and the right displacement platform (12), the left displacement platform (3) and the right displacement platform (12) are respectively set on opposite sides of the housing (14), the left mirror (5) and the right mirror (11) are inclined, the cameras (8) are in front of the left mirror (5) and the right mirror (11), the near-infrared light source (9) is located below the cameras (8), and the visible light columns (13) are arranged in multiple columns and configured longitudinally along an inner side of the housing (14).

2. The apparatus in accordance with claim 1, wherein there are two cameras (8), which are respectively located in front of the left mirror (5) and the right mirror (11).

3. The apparatus in accordance with claim 1, wherein each column of the visible light columns (13) is composed of one or more discrete point light sources.

4. A method for evaluating exophthalmos, using the apparatus according to any one of Claims 1 to 3, comprising the following steps:
Step S1: Adjust and fix the lateral canthus; controlling the near-infrared light source to light up a plurality of columns of visible light columns in sequence; recording, by means of the camera, a video of the eyeball looking straight ahead; acquiring, from the video, a video frame when the longest reflected light of the visible light columns appears on the eyeball in the mirrors;
Step S2: Determine a corneal vertex from the video frame, and using a neural network to determine a pupil center;
Step S3: Calculate the ocular prominence on the basis of the known position, the inclination angle of the mirror, the corneal vertex, and the pupil center;
wherein the known position is a position of the optical center of the camera, the distance between the optical center of the camera and the frame of the video, and the position of the lateral canthus, and the inclination angle of the mirror is the included angle between the mirror and an imaging surface.

5. The method in accordance with Claim 4, wherein in Step S1, to light up a plurality of columns of visible light columns in sequence, specifically meaning that the visible light columns are illuminated sequentially from the middle to the outside, or from the outside to the middle.

6. The method in accordance with Claim 5, further comprising when the visible light column on one side is illuminated, a video of the opposite eye is captured.

7. The method in accordance with Claim 4, wherein, in Step S3, the optical center is O, the lateral canthus is E, the distance between the optical center O and the frame of the video is OB, an imaging point of a corneal vertex Z on the frame of the video is C, and a reflection point U of the corneal vertex Z in the mirror is an imaging point A on the video frame; the orthogonal distance from the imaging point C and the imaging point A to an optical axis are AB and CB, respectively, the inclination angle between the mirror and the eye is ∠ UED, the distance from the optical center O to a face plane of the lateral canthus E is OF, a distance between the lateral canthus E and the optical axis is EF, the ocular prominence is ZG which is a orthogonal distance between the corneal vertex Z and a straight line DE; each of OB, OF, EF, AB, and CB has a known length, and the inclination angle ∠UED is known, and Step S3 comprises:
Step S31: The line passing through the reflection point U of the corneal vertex Z in the mirror and parallel to the straight line DE intersects with the optical axis at a point W, with ZG at a point Y, and with an extension of a line OC at a point X;
Step S32: Find a length of a line segment WF: $WF = \frac{OFtan ∠ UOW - EF}{tan ∠ UOW + \frac{1}{tan ∠ UED}}$
Step S33: Using iterative calculation to obtain a length of a line segment ZY; and
Step S34: calculate the ocular prominence according to ZG = WF + ZY.

8. An evaluation system for implementing the method according to any one of Claim 4 to 7, wherein the system includes:
a video acquisition module, configured to acquire the video of the eyeball looking straight ahead when the multiple visible light columns from the near-infrared light source light up in sequence;
an image acquisition module, configured to obtain the video frame having the longest reflected light from the video;
a feature extraction module, configured to extract the corneal vertex and the pupil center from the video frame; and
a calculation module, configured to calculate the ocular prominence from the known position and the mirror inclination angle, the corneal vertex, and the pupil center, wherein the known position is the position of the optical center of the camera, the distance between the optical center of the camera and the video frame, and the position of the lateral canthus, and the inclination angle of the mirror is the included angle between the mirror and the imaging surface.

9. A computer-readable storage medium, storing at least one program code adapted to be loaded and executed by a processor to perform the method according to any one of Claim 4 to 7.

## Patentansprüche

1. Vorrichtung zur Bewertung des Exophthalmus für ein Exophthalmus-Untersuchungsgerät, **dadurch gekennzeichnet, dass** sie einen linken Verstelltisch (3), einen linken Augenwinkel-Anschlagpunkt (4), einen linken Spiegel (5), eine Kamera (8), eine Nahinfrarotlichtquelle (9), einen rechten Augenwinkel-Anschlagpunkt (10), einen rechten Spiegel (11), einen rechten Verstelltisch (12), sichtbare Lichtsäulen (13) und ein Gehäuse (14) umfasst; wobei der linke Spiegel (5) und der rechte Spiegel (11) jeweils an dem linken Augenwinkel-Anschlagpunkt (4) bzw. dem rechten Augenwinkel-Anschlagpunkt (10) angeordnet sind, der linke Augenwinkel-Anschlagpunkt (4) und der rechte Augenwinkel-Anschlagpunkt (10) jeweils auf dem linken Verstelltisch (3) bzw. dem rechten Verstelltisch (12) angeordnet sind, der linke Verstelltisch (3) und der rechte Verstelltisch (12) jeweils an beiden Seiten des Gehäuses (14) angeordnet sind, und der linke Spiegel (5) und der rechte Spiegel (11) beide geneigt angeordnet sind; wobei sich die Kamera (8) vor dem linken Spiegel (5) und dem rechten Spiegel (11) befindet; wobei sich die Nahinfrarotlichtquelle (9) unterhalb der Kamera (8) befindet; wobei die sichtbaren Lichtsäulen (13) mehrreihig ausgebildet und entlang der Innenseite des Gehäuses (14) in Längsrichtung verteilt angeordnet sind.

2. Vorrichtung zur Bewertung des Exophthalmus für ein Exophthalmus-Untersuchungsgerät nach Anspruch 1, **dadurch gekennzeichnet, dass** die Kamera (8) als zwei Kameras ausgebildet ist, die sich jeweils vor dem linken Spiegel (5) und dem rechten Spiegel (11) befinden.

3. Vorrichtung zur Bewertung des Exophthalmus für ein Exophthalmus-Untersuchungsgerät nach Anspruch 1, **dadurch gekennzeichnet, dass** jede Reihe der sichtbaren Lichtsäulen (13) aus diskreten Punktlichtquellen besteht.

4. Verfahren zur Bewertung des Exophthalmus, **dadurch gekennzeichnet, dass** es die Vorrichtung zur Bewertung des Exophthalmus für ein Exophthalmus-Untersuchungsgerät nach einem der Ansprüche 1 bis 3 verwendet und die folgenden Schritte umfasst:
Schritt S1: Einstellen und Fixieren des lateralen Augenwinkelpunkts (äußerer Kanthus), sequenzielles Aufleuchtenlassen der mehrreihigen sichtbaren Lichtsäulen durch Steuern der Nahinfrarotlichtquelle, Aufnehmen eines Videos des geradeaus blickenden Augapfels mittels einer Kamera, und Erfassen des Videobildes aus dem Video, in dem der längste Reflex des sichtbaren Lichtsäulenlichts auf dem Augapfel im Spiegel erscheint;
Schritt S2: Bestimmen des Hornhautscheitels aus dem Videobild und Bestimmen der Pupillenmitte mittels eines neuronalen Netzes;
Schritt S3: Berechnen des Exophthalmus anhand der bekannten Positionen, des Neigungswinkels des Spiegels, des Hornhautscheitels und der Pupillenmitte;
wobei die bekannten Positionen die Position des optischen Zentrums der Kamera, der Abstand des optischen Zentrums der Kamera zum Videobild und die Position des lateralen Augenwinkelpunkts umfassen, und der Neigungswinkel des Spiegels der Winkel zwischen Spiegel und Bildebene ist.

5. Verfahren zur Bewertung des Exophthalmus nach Anspruch 4, **dadurch gekennzeichnet, dass** in dem Schritt S1 die mehrreihigen sichtbaren Lichtsäulen sequenziell aufleuchten, insbesondere indem sie von der Mitte nach außen oder von außen zur Mitte hin sequenziell aufleuchten.

6. Verfahren zur Bewertung des Exophthalmus nach Anspruch 5, **dadurch gekennzeichnet, dass** in dem Schritt S1, wenn die sichtbaren Lichtsäulen auf einer Seite aufleuchten, ein Video des Auges auf der gegenüberliegenden Seite aufgenommen wird.

7. Verfahren zur Bewertung des Exophthalmus nach Anspruch 4, **dadurch gekennzeichnet, dass** in dem Schritt S3 das optische Zentrum O ist, der laterale Augenwinkelpunkt E ist, der Abstand vom optischen Zentrum O zu dem Videobild OB ist, der Abbildungspunkt des Hornhautscheitels Z auf dem Videobild C ist, der Abbildungspunkt des Reflexionspunktes U des Hornhautscheitels Z im Spiegel auf dem Videobild A ist, wobei der senkrechte Abstand des Abbildungspunkts C von der optischen Achse CB und der des Abbildungspunkts A von der optischen Achse AB ist, der Neigungswinkel zwischen Spiegel und Auge ∠UED ist, der Abstand vom optischen Zentrum O zu der Gesichtsebene, in der der laterale Augenwinkelpunkt E liegt, OF ist, der Abstand vom lateralen Augenwinkelpunkt E zu der optischen Achse EF ist, der Exophthalmus der senkrechte Abstand ZG vom Hornhautscheitel Z zur Geraden DE ist, wobei die Längen von OB, OF, EF, AB, CB und der Winkel ∠UED bekannt sind; wobei der Schritt S3 umfasst:
Schritt S31: Eine Gerade, die durch den Reflexionspunkt U des Hornhautscheitels Z im Spiegel verläuft und parallel zur Geraden DE ist, schneidet die optische Achse im Punkt W, die Strecke ZG im Punkt Y und die Verlängerung der Geraden OC im Punkt X;
Schritt S32: Bestimmen der Länge der Strecke WF: $WF = \frac{OFtan ∠ UOW - EF}{tan ∠ UOW + \frac{1}{tan ∠ UED}} ;$
Schritt S33: Iteratives Berechnen der Länge der Strecke ZY;
Schritt S34: Der Exophthalmus beträgt: *ZG=WF+ZY.*

8. Bewertungssystem zur Durchführung des Verfahrens zur Bewertung des Exophthalmus nach einem der Ansprüche 4 bis 7, **dadurch gekennzeichnet, dass** das System umfasst:
ein Videoerfassungsmodul, das dazu dient, beim sequenziellen Aufleuchten der mehrreihigen sichtbaren Lichtsäulen der Nahinfrarotlichtquelle ein Video des geradeaus blickenden Augapfels zu erfassen;
ein Bildgewinnungsmodul, das dazu dient, aus dem Video das Videobild zu gewinnen, in dem der Reflex des sichtbaren Lichtsäulenlichts im Spiegel am längsten ist;
ein Merkmalsextraktionsmodul, das dazu dient, aus dem Videobild den Hornhautscheitel und die Pupillenmitte zu extrahieren;
ein Berechnungsmodul, das dazu dient, anhand der bekannten Positionen, des Neigungswinkels des Spiegels, des Hornhautscheitels und der Pupillenmitte den Exophthalmus zu berechnen; wobei die bekannten Positionen die Position des optischen Zentrums der Kamera, der Abstand des optischen Zentrums der Kamera zum Videobild und die Position des lateralen Augenwinkelpunkts umfassen, und der Neigungswinkel des Spiegels der Winkel zwischen Spiegel und Bildebene ist.

9. Computerlesbares Speichermedium, **dadurch gekennzeichnet, dass** in dem computerlesbaren Speichermedium mindestens ein Programmcode gespeichert ist, wobei der mindestens eine Programmcode von einem Prozessor geladen und ausgeführt wird, um das Verfahren zur Bewertung des Exophthalmus nach einem der Ansprüche 4 bis 7 durchzuführen.

## Revendications

1. Dispositif d'évaluation de l'exophtalmie pour un exophtalmomètre, **caractérisé en ce qu'** il comprend : une plateforme de déplacement gauche (3), un point de repère du canthus latéral gauche (4), un miroir gauche (5), une caméra (8), une source lumineuse proche infrarouge (9), un point de repère du canthus latéral droit (10), un miroir droit (11), une plateforme de déplacement droit (12), des barres lumineuses en lumière visible (13), un boîtier (14) ; ledit miroir gauche (5) et ledit miroir droit (11) sont respectivement disposés sur ledit point de repère du canthus latéral gauche (4) et sur ledit point de repère du canthus latéral droit (10), lesdits points de repère du canthus latéral gauche (4) et du canthus latéral droit (10) étant respectivement disposés sur ladite plateforme de déplacement gauche (3) et sur ladite plateforme de déplacement droit (12), lesdites plateformes de déplacement gauche (3) et droite (12) étant respectivement disposées sur les deux côtés dudit boîtier (14), ledit miroir gauche (5) et ledit miroir droit (11) étant tous deux inclinés ; ladite caméra (8) est située en avant desdits miroirs gauche (5) et droit (11) ; ladite source lumineuse proche infrarouge (9) est située en dessous de ladite caméra (8) ; lesdites barres lumineuses en lumière visible (13) sont disposées en plusieurs rangées et réparties longitudinalement le long de la face interne dudit boîtier (14).

2. Dispositif d'évaluation de l'exophtalmie pour un exophtalmomètre selon la revendication 1, **caractérisé en ce qu'** il comporte deux caméras (8) situées respectivement en avant dudit miroir gauche (5) et dudit miroir droit (11).

3. Dispositif d'évaluation de l'exophtalmie pour un exophtalmomètre selon la revendication 1, **caractérisé en ce que** chaque rangée desdites barres lumineuses en lumière visible (13) est constituée de sources lumineuses ponctuelles discrètes.

4. Procédé d'évaluation de l'exophtalmie, **caractérisé en ce qu'** il met en œuvre le dispositif d'évaluation selon l'une quelconque des revendications 1 à 3, comprenant les étapes suivantes :
Étape S1 : ajuster et fixer le point du canthus latéral, commander la source lumineuse proche infrarouge pour faire s'allumer successivement les multiples barres lumineuses en lumière visible, acquérir une vidéo de l'œil regardant droit devant au moyen de la caméra, et extraire de ladite vidéo l'image vidéo dans laquelle la réflexion la plus longue des barres lumineuses en lumière visible apparaît sur l'œil dans le miroir ;
Étape S2 : déterminer le sommet cornéen à partir de ladite image vidéo, et déterminer le centre de la pupille au moyen d'un réseau neuronal ;
Étape S3 : calculer l'exophtalmie à partir de positions connues, de l'angle d'inclinaison du miroir, dudit sommet cornéen et dudit centre de la pupille ;
ladite position connue étant la position du centre optique de la caméra, la distance du centre optique de la caméra à l'image vidéo, et la position du point du canthus latéral, ledit angle d'inclinaison du miroir étant l'angle entre le miroir et le plan d'imagerie.

5. Procédé d'évaluation de l'exophtalmie selon la revendication 4, **caractérisé en ce que**, à ladite étape S1, lesdites multiples barres lumineuses en lumière visible s'allument successivement, en particulier du centre vers l'extérieur, ou de l'extérieur vers le centre.

6. Procédé d'évaluation de l'exophtalmie selon la revendication 5, **caractérisé en ce que**, à ladite étape S1, lorsqu'une barre lumineuse en lumière visible d'un côté s'allume, la vidéo de l'œil du côté controlatéral est enregistrée.

7. Procédé d'évaluation de l'exophtalmie selon la revendication 4, **caractérisé en ce que**, à ladite étape S3, le centre optique est O, le point du canthus latéral est E, la distance du centre optique O à ladite image vidéo est OB, le point d'imagerie du sommet cornéen Z sur ladite image vidéo est C, le point d'imagerie sur ladite image vidéo du point de réflexion U du sommet cornéen Z dans le miroir est A, les distances perpendiculaires des points d'imagerie C et A à l'axe optique sont respectivement AB et CB, l'angle d'inclinaison du miroir par rapport à l'œil est ∠UED, la distance du centre optique O au plan facial contenant ledit point du canthus latéral E est OF, la distance du point du canthus latéral E audit axe optique est EF, l'exophtalmie est la distance perpendiculaire ZG du sommet cornéen Z à la ligne DE, les longueurs OB, OF, EF, AB, CB et l'angle ∠UED étant connus ; ladite étape S3 comprend :
Étape S31 : la ligne passant par le point de réflexion U du sommet cornéen Z dans le miroir et parallèle à la ligne DE coupe l'axe optique au point W, coupe ZG au point Y, et coupe le prolongement de la ligne OC au point X ;
Étape S32 : déterminer la longueur du segment WF : $WF = \frac{OFtan ∠ UOW - EF}{tan ∠ UOW + \frac{1}{tan ∠ UED}} ;$
Étape S33 : déterminer la longueur du segment ZY par calcul itératif ;
Étape S34 : l'exophtalmie est : *ZG=WF+ZY*.

8. Système d'évaluation pour la mise en œuvre du procédé d'évaluation de l'exophtalmie selon l'une quelconque des revendications 4 à 7, **caractérisé en ce que** ledit système comprend :
un module d'acquisition vidéo, configuré pour acquérir une vidéo de l'œil regardant droit devant lorsque les multiples barres lumineuses en lumière visible de la source lumineuse proche infrarouge s'allument successivement ;
un module d'extraction d'image, configuré pour extraire de ladite vidéo l'image vidéo dans laquelle la réflexion des barres lumineuses en lumière visible dans le miroir est la plus longue ;
un module d'extraction de caractéristiques, configuré pour extraire le sommet cornéen et le centre de la pupille à partir de ladite image vidéo ;
un module de calcul, configuré pour calculer l'exophtalmie à partir de positions connues, de l'angle d'inclinaison du miroir, dudit sommet cornéen et dudit centre de la pupille ; ladite position connue étant la position du centre optique de la caméra, la distance du centre optique de la caméra à l'image vidéo, et la position du point du canthus latéral, ledit angle d'inclinaison du miroir étant l'angle entre le miroir et le plan d'imagerie.

9. Support de stockage lisible par ordinateur, **caractérisé en ce que** ledit support de stockage lisible par ordinateur contient au moins un code de programme, ledit au moins un code de programme étant chargé et exécuté par un processeur pour mettre en œuvre le procédé d'évaluation de l'exophtalmie selon l'une quelconque des revendications 4 à 7.
